Europäisches Patentamt

(19) European Patent Office

Office européen des brevets

(11) EP 0 781 506 A1

(12) **EUROPEAN PATENT APPLICATION**
published in accordance with Art. 158(3) EPC

(43) Date of publication:
02.07.1997 Bulletin 1997/27

(21) Application number: 94925019.5

(22) Date of filing: 29.08.1994

(51) Int. Cl.$^6$: **A01K 67/027**

(86) International application number:
PCT/JP94/01422

(87) International publication number:
WO 96/06525 (07.03.1996 Gazette 1996/11)

(84) Designated Contracting States:
AT BE CH DE DK ES FR GB GR IE IT LI LU MC NL
PT SE

(71) Applicant: TORII PHARMACEUTICAL CO., LTD.
Tokyo 103 (JP)

(72) Inventors:
• SHINOHARA, Masami
Koshatawa Komatsugawa 1802
Tokyo 132 (JP)
• USHIJIMA, Taro
Chiba 267 (JP)
• MASUYAMA, Taku
Chiba 267 (JP)

(74) Representative: O'Brien, Caroline Jane et al
MEWBURN ELLIS
York House
23 Kingsway
London WC2B 6HP (GB)

(54) **SPONTANEOUSLY DIABETIC RAT**

(57) The present invention relates to diabetic rats established from the Sprague-Dawley (SD) strain.

**Description**

FIELD OF INDUSTRIAL APPLICABILITY

The present invention relates to spontaneously diabetic rats which can be utilized for the development of therapeutic agents for diabetes and the studies on treatment of diabetes.

PRIOR ART

For the studies relating to the diseases presenting a specific state of affection and therapeutics for such diseases, utilization of the experimental animals developing the same state of ailment is essential. Hitherto, in this line of studies, many efforts have been directed to producing the diseased animals by giving drugs to the normal ones or performing operations on them, or to finding and utilizing the animals which have developed the particular morbid state mutagenically and transmitted it genetically and which are capable of propagation.

As the spontaneously diabetic animals available for the studies of diabetes, there have been known WBN/Kob rat (1), GK rat (2), BB rat (3,4), KK mouse (5) and NOD mouse (6) and the like. In the experiments of efficacy of therapeutic agents in which diabetes was caused artificially by medication, the drug (Streptozotocin, Alloxan, etc.)-induced diabetic rats have been popularly used. However, the spontaneously diabetic animals available at present are unsatisfactory in such respects as breeding potential, rate of occurrence of a particular disease, time required till the desired morbid state is produced and occurrence of a complication similar to that observed in human. Also, in the drug-induced diabetic rats, the mechanism of occurrence of the disease is different from the ordinary diabetes, so that they don't necessarily offer a reflection of the human diabetes.

(1) Mori et al: J. Jpn. Diabetes Soc., Vol. 31, No. 1, 15-19, 1988.
(2) Goto et al: Proc. Jpn. Acad., 51, 80-85, 1975.
(3) Chappel, C.I. et al: Metabolism, 32, (7) Suppl. 1, pp. 9-10, 1983.
(4) Kawazu et al: Infection, Inflammation & Immunity, Vol. 15, No. 1, 28-39, 1985.
(5) Matsuo, T. et al: J. Takeda Res. Lab., 30, 307-, 1971.
(6) Koike et al: Infection, Inflammation & Immunity, Vol. 17, No. 4, 28-38, 1987.

PROBLEMS TO BE SOLVED BY THE INVENTION

It is accordingly an object of the present invention to eliminate the above problems and to establish a morbid model rat which is easy to handle and fertile, has a high rate of occurrence of diabetes, needs a short period of time till occurrence of diabetes and develops a characteristic diabetic complication similar to that seen in human.

MEANS FOR SOLVING THE PROBLEMS

In the present invention, the above problems have been solved by the non-obese diabetic rats established from the Sprague-Dawley (SD) strain. That is, the diabetic rats were established by using as seed parent the male SD rats affected by diabetes and repeating sib mating with uterine female rats. The diabetic rats of the present invention were named Spontaneously Diabetic strain-Torii (SDT) rats by the present inventors and are referred to by this name in the present specification of the invention.

1. Origin

In 1988, it was learned that in a group of SD rats breeded over a long time in an animal breeding room in the laboratory of Torii Pharmaceutical Co., Ltd., there were some rats which voided sweet-smelling urine in volume, and as a result of careful observation, 5 spontaneously diabetic rats were detected from 305 male rats in the group. Using these spontaneously diabetic rats as origin, the present inventors launched on rearing of the strain which develops diabetes at a high rate by repeating sib mating of these rats.

2. Reproduction

SDT rats are capable of propagation by sib mating, have high breeding potential and show no abnormality in nursing behavior (by female rats) nor unbalance of sex ratio. They throw a litter of 6 to 8 at one delivery, and the weanling rate is 80-90%. Each female has a fertility capability of delivery of up to 2-3 times.

2

3. Maintenance of strain

For the maintenance of the strain, there is employed a mating method in which the diabetic male rats and the non-diabetic female rats are picked up at random from the uterine brother and sister rats, with 1-2 female rats being allowed to reside with one male rat to let them repeat sib mating. With this method, it is possible to maintain the strain of SDT rats and to obtain the next generation at a constant rate.

4. Characteristics of morbid state

The morbid state of SDT rats is characterized by the following specificities:

1) Male rats alone contract diabetes, showing an obvious difference between sexes regarding onset of diabetes.
2) SDT rats are smaller in body than the normal rats even before contracting diabetes. They loss weight and show no obesity after contracting diabetes.
3) Diabetes of SDT rats occurs relatively suddenly. Severe positivity in glycosuria is observed at the time of onset of diabetes, and thereafter urinary volume, water intake and food consumption increase.
4) Onset of diabetes is seen from 2.5th month after birth, the average age of onset of diabetes being about 4 months after birth. The incidence of diabetes is 90% or more and very stabilized.
5) Glucose and glycated hemoglobin level in blood rises while insulin level in blood decreases with progress of diabetes.
6) SDT rats can survive more than one year after onset of diabetes even if they have no insulin treatment.
7) Cataracts
   The crystalline lens of the eyeball becomes cloudy as the diabetic condition worsens. The degree of cloudiness increases sharply at 3 to 4 months after onset of diabetes. Cataracts are seen in all cases.
8) Patho-histological observation
   The patho-histological peculiarities of SDT rats area trophy or disappearance of the islets of Langerhans of the pancreas, hyperplasia of pancreatic ducts and dilation of renal tubules.

5. Environment for breeding and reproduction

SDT rats were housed in a stainless steel-made wire mesh cage (mfd. by Toyo Riko KK) and reared under an SPF environment adjusted to 23±2°C and 55 ±5% Relative Humidity and illuminated with lamps for 12 hours, the lamps being lit up at 8 am and put out at 8 pm. The rats were allowed to take solid feed (CE-2 available from Clea Ltd., Japan) and tap water ad-lib. However, during the breeding period (the period from birth to ablactation), the rats were reared in a polycarbonate cage (280W x 440D x 180H available from Toyo Riko KK) where a proper amount of flooring (White Flake mfd. by Charles River Japan, Inc.) was placed.

6. Confirmation of onset of diabetes

Confirmation of onset of diabetes of these rats was made by forcibly letting them urinate at a rate of three times a week and examining the urine using an urine testing paper (MULTI-STICKS available from Mailes Sankyo, Inc.). The above test was conducted whenever there was noticed a possibility of onset of diabetes from the state of fouling or smell of the soil tray or other abnormal symptoms such as increase of water intake during daily care routine. The individual whose glycosuric test result proved positive was determined to be a diabetes-affected individual, and the date of this detection was designated the date of onset diabetes.

By employing the above rearing conditions and test method, the present inventors have succeeded in creating the morbid model rats of spontaneous non-obese diabetes of the present invention from Sprague-Dawley (SD) rats, said morbid model rats being similar to human diabetic state with no obesity and having a high incidence of diabetes.

EFFECT OF THE INVENTION

The rats provided according to the present invention present various symptoms of diabetes such as severe positivity of glycosuria and hiperglycemia at about 4 months after birth and develop complications such as cataracts, so that these rats are useful as experimental animals for the studies on diabetes and their therapeutic agents.

EXAMPLES

The diabetic rats of the present invention were established by repeating sib mating, and the incidence of diabetes in the male rats is 90% or more. For illustrating the present invention more particularly, a comparison between the rats of the present invention and the normal rats is given below.

1. Test for diabetes

The following test was carried out using five 6-month-old male SDT rats which contracted diabetes at the age of 3 months after birth and five male Sprague-Dawley (SD) rats of the same age. Using the obtained results, a statistically significant difference examination by Student's T-Test was conducted.

① Measurement of blood-glucose level

The blood-glycose level was measured by an enzyme method using glucose oxidase and a glucose measuring kit (Glucose B Test-Wako mfd. by Wako Pure Chemicals Co., Ltd.). Heparinized blood was collected from each test rat and immediately centrifuged, and the supernatant (plasma) was used as test specimen. The color reagent (containing glucose oxidase, peroxidase and 4-aminoantipyrine) in the kit was dissolved in a buffer solution (30 mM phosphate buffer, pH 7.4) to prepare a color developing solution. 3.0 ml of this color developing solution was mixedwell with 0.02 ml of specimen and kept at 37°C for 20 minutes, after which absorbance at 505 nm was measured, with blank test reagent (color developing solution alone) as control. Calibration curves were drawn up simultaneously for 50-500 mg/dl of glucose, and the glucose concentration of the test specimen was calculated therefrom.

The results obtained from the above method are shown in Table 1.

Table 1

| Animal | | Blood-glucose level mg/dl |
|---|---|---|
| Control SD rat | 1 | 130.7 |
| | 2 | 123.3 |
| | 3 | 108.9 |
| | 4 | 132.9 |
| | 5 | 131.0 |
| Mean value ± standard error | | 125.4 ± 4.43 |
| SDT rat | 1 | 601.6 |
| | 2 | 535.8 |
| | 3 | 663.7 |
| | 4 | 717.4 |
| | 5 | 573.3 |
| Mean value ± standard error | | 618.4 ± 32.4 |
| Statistically significant difference $p < 0.001$ | | |

As is seen from Table 1, SDT rats showed apparently higher blood-glucose level than control SD rats.

② Measurement of glycated hemoglobin

Measurement of glycated hemoglobin (glycohemoglobin) was made using a glycated hemoglobin measuring kit (Glycohemoglobin Test-Wako mfd. by Wako Pure Chemicals Co., Ltd.). Heparinated blood collected from each test rat was immediately centrifuged to remove plasma and leukocytes. To the resulting enthroid fraction was added a cold physiological saline at a rate of 3 (saline) to 1 (enthroid fraction) in volume, and they were mixed by tumbling down and centrifuged (at 1,500 r.p.m. for 10 minutes). To 0.1 ml of the thus obtained enthrocytes was added 2.0 ml of distilled water, followed by stirring by a mixer for 15-20 seconds and centrifuging. The sediments (red blood cell membranes, residual blood corpuscles, etc.) were removed and the supernatant was collected as hemolytic solution and kept at -80°C until measurement was made. Measurement of glycated hemoglobin levels was made according to affinity chromatography using agarose in which m-aminophenylboronic acid bonded to glucose contained in glycated hemoglyobin has been fixed. More specifically, 0.05 ml of said hemolytic solution was added to a separating column in a kit which had previously been washed with 2 ml of a buffer solution (0.25M ammonium acetate buffer, pH 8.0, containing 50 mmol/l of magnesium chloride), and then 0.5 ml of said buffer solution was added to move the hemolytic solution into the column. Thereafter, 5 ml of the buffer solution was further added to cause efflux and the effluent was collected as non-glycated hemoglobin fraction. 3.0 ml of an eluting buffer solution (0.1M tris buffer solution with pH 8.0, containing 0.2 mmol/l of sorbitol) was added to the same column and the eluate was collected as glycated homoglobin fraction.

Absorbance (at 414 nm) of said two fractions was measured by a spectrophotometer, with distilled water as control, and using the obtained values, glycated homoglobin concentration was calculated from the following equation:

Glycated hemoglobin % = 3.0 x absorbance at 414 nm (glycated hemoglobin)/(5.55 x absorbance at 414 nm (non-glycated hemoglobin) + 3.0 x absorbance at 414 nm (glycated hemoglobin)) x 100

Glycohemoglobin Control mfd. by Pierce Chemical Company (U.S.) was used as standard for measurement. The results obtained from the above method are shown in Table 2.

Table 2

| Animal | | Glycated hemoglobin (%) |
|---|---|---|
| Control SD rat | 1 | 2.9 |
| | 2 | 4.4 |
| | 3 | 3.9 |
| | 4 | 2.1 |
| | 5 | 2.0 |
| Mean value ± standard error | | 3.1 ± 0.48 |
| SDT rat | 1 | 18.7 |
| | 2 | 16.6 |
| | 3 | 22.7 |
| | 4 | 22.4 |
| | 5 | 19.5 |
| Mean value ± standard error | | 20.0 ± 1.15 |
| Statistically significant difference p < 0.001 | | |

As is seen from Table 2, an apparent increase of glycated hemoglobin is noted in SDT rats as compared with control SD rats, which agrees with clinical observation of diabetes in human. This indicates that SDT rats show the progress of diabetes similar to that in man, and suggests usefulness of SDT rats for the studies on advance of the diabetic state.

③ Measurement of blood insulin

Blood insulin was measured by radioimmuniassay (RIA).

The blood collected from each test rat was put into a test tube containing a serum separating agent and centrifuged at 3,000 r.p.m. for 30 minutes, and the supernatant (serum) was assayed. To 0.1 ml of test serum were added 0.1 ml of guinea pig anti-human insulin antiserum and 0.1 ml of a phosphate buffer (50 mM, pH 7.4) containing 2% bovine serumal bumin and 10 mM EDTA, followed by addition of a labeling medium (a solution of human insulin, 125I-labeled at Tyr A14 and IM116, adjusted to 10,000 cpm/$\mu$l with a phosphate buffer), and the mixture was left at 4°C for 20 minutes. To the resulting reaction solution was added 0.25 ml of a dextran/activated carbon mixed solution (a solution obtained by mixing 0.8 g of activated carbon and dextran and stirring the mixture with 50 ml of a buffer solution at 4°C for 20 minutes), and the mixture was centrifuged at 3,000 r.p.m. at 4°C for 20 minutes. The supernatant was pipetted into each test tube, and after measurement by a $\gamma$-ray counter for 60 seconds, the serum insulin level was calculated from the simultaneously drawn up calibration curve.

The results obtained from the above process are shown in Table 3.

Table 3

| Animal | | Serum insulin ($\mu$U/ml) |
|---|---|---|
| Control SD rat | 1 | 46.1 |
| | 2 | 31.0 |
| | 3 | 42.3 |
| | 4 | 81.6 |
| | 5 | 16.7 |
| Mean value $\pm$ standard error | | 43.5 $\pm$ 10.8 |
| SDT rat | 1 | 11.7 |
| | 2 | 2.2 |
| | 3 | 3.1 |
| | 4 | 9.3 |
| | 5 | 4.6 |
| Mean value $\pm$ standard error | | 6.2 $\pm$ 1.84 |
| Statistically significant difference $p < 0.01$ | | |

As is seen from Table 3, the blood insulin of SDT rats was lower than that of control SD rats, suggesting that SDT rats were affected by hypoinsulinemia.

④ Examination of urine

Urinary examination was made by keeping the test animals in a stainless steel-made metabolic cage in a well-fed state for 24 hours, collecting their urine by a urine collecting pot and analyzing the collected urine. The amount of urine was measured by a graduated cylinder, and then a qualitative examination of urine was conducted using urine testing paper (MULTI-STICKS mfd. by Mailes Sankyo, Inc.). Part of the collected urine was centrifuged at 3,000 r.p.m. for 10 minutes and the supernatant was offered to assay of urinary protein. Urinary protein was assayed by Kingsbury-Clark method in which 3.0 ml of 3% sulfosalicylic acid solution was added to 1 ml of sample urine and mixed, and absorbance of the solution at a wavelength of 660 nm was measured. Concentration was also determined from the simultaneously drawn up calibration curve. The results obtained from the above operations are shown in Table 4 regarding the amount of urine, Table 5 regarding the results of the test with urine testing paper and Table 6 regarding the amount of urinary protein excreted.

Table 4

| Animal | | Urinary volume (ml) |
|---|---|---|
| Control SD rat | 1 | 16 |
| | 2 | 8 |
| | 3 | 14 |
| | 4 | 20 |
| | 5 | 14 |
| Mean value ± standard error | | 14.4 ± 1.94 |
| SDT rat | 1 | 70 |
| | 2 | 75 |
| | 3 | 50 |
| | 4 | 50 |
| | 5 | 35 |
| Mean value ± standard error | | 56.0 ± 7.31 |
| Statistically significant difference $p < 0.001$ | | |

Table 5

| Animal | | Protein | Ketone body | Dextrose |
|---|---|---|---|---|
| Control SD rat | 1 | ± | ± | - |
| | 2 | ± | ± | - |
| | 3 | ± | - | - |
| | 4 | + | ± | - |
| | 5 | + | ± | - |
| SDT rat | 1 | ± | - | 4+ |
| | 2 | ± | - | 4+ |
| | 3 | + | ± | 4+ |
| | 4 | 2+ | - | 4+ |
| | 5 | 2+ | ± | 4+ |
| Note: All cases were negative (-) in urobilinogen, occult blood and bilirubin reaction tests. | | | | |

Table 6

| Animal | | Amount of urinary protein excreted (mg/day) |
|---|---|---|
| Control SD rat | 1 | 3.9 |
| | 2 | 3.6 |
| | 3 | 3.3 |
| | 4 | 5.5 |
| | 5 | 4.8 |
| Mean value ± standard error | | 4.2 ± 0.41 |
| SDT rat | 1 | 17.9 |
| | 2 | 10.3 |
| | 3 | 11.4 |
| | 4 | 24.2 |
| | 5 | 30.2 |
| Mean value ± standard error | | 18.8 ± 3.79 |
| Statistically significant difference p < 0.01 | | |

As is seen from Table 4 and Table 5, all of the SDT rats tested were polyuric and strongly positive in glycosuria reaction test, and gave the apparently different results from control SD rats. Thus, the rats of the present invention are typified by polyuria and excretion of glycosuria. Also, as shown in Table 6, the amount of urinary protein excreted by SDT rats per day was significantly greater than that by control SD rats. In view of the fact that, in SDT rats, the above phenomena are not observed before onset of diabetes or in the early phase of contraction, it is expected that SDT rats which have had about 3 months of diabetic contraction would present a morbid state resembling that of diabetic renal injury in human, which suggests that these SDT rats would be suited for studies not only on such diabetic symptons as consistent hyperglycemia and positivity in glycosuria but also on diabetic renal injury, morbid patho-physiological functions and such matters.

**Claims**

1. Diabetic rats established from the Sprague-Dawley (SD) strain.

2. The diabetic rats according to Claim 1, which show difference in contraction of diabetes according to the sex, with the male rats alone contracting diabetes.

3. The diabetic rats according to Claim 1 or 2, which develop diabetic complications such as cataracts at 3-4 months from onset of diabetes.

4. The diabetic rats according to Claim 1 or 2, which can survive for a long time even after onset of diabetes, have high fertility and are capable of genetic transfer for generations.

5. The diabetic rats according to any one of Claims 1-4, wherein the Sprague-Dawley (SD) strain rats have been established by using the diabetes-affected male rats as seed rats, and repeating sib mating with the uterine female rats.

6. A method for creating the diabetic rats set forth in any one of Claims 1-5 which comprises using diabetes-contracted Sprague-Dawley (SD) male rats as seed rats, and repeating sib mating with the uterine female rats.

# INTERNATIONAL SEARCH REPORT

| International application No. |
|---|
| PCT/JP94/01422 |

**A. CLASSIFICATION OF SUBJECT MATTER**

Int. Cl$^6$   A01K67/027

According to International Patent Classification (IPC) or to both national classification and IPC

**B. FIELDS SEARCHED**

Minimum documentation searched (classification system followed by classification symbols)

Int. Cl$^5$   A01K67/027

Documentation searched other than minimum documentation to the extent that such documents are included in the fields searched

Electronic data base consulted during the international search (name of data base and, where practicable, search terms used)

BIOSIS PREVIEWS

**C. DOCUMENTS CONSIDERED TO BE RELEVANT**

| Category* | Citation of document, with indication, where appropriate, of the relevant passages | Relevant to claim No. |
|---|---|---|
| X | JP, A, 4-252129 (Otsuka Pharmaceutical Co., Ltd.), | 1, 5, 6 |
| Y | September 8, 1992 (08. 09. 92), (Family: none) | 2, 3, 4 |
| X | JP, A, 3-266926 (Otsuka Pharmaceutical Co., Ltd.), | 1 |
| Y | November 27, 1991 (27. 11. 91), (Family: none) | 2-6 |
| A | Mineral and Electrolyte Metabolism Vol. 10, No. 3, (1984) Vaamonde C.A. et al "Effect of Duration of Diabates on the Protection observed in the Diabetic rat against Gentamicin induced acute renal Failure" | 1-6 |

☐ Further documents are listed in the continuation of Box C.     ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| November 4, 1994 (04. 11. 94) | November 29, 1994 (29. 11. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)